**Europäisches Patentamt**

**European Patent Office**

**Office européen des brevets**

(19)

(11) Veröffentlichungsnummer: **0 337 239**
**A2**

# (12) EUROPÄISCHE PATENTANMELDUNG

(21) Anmeldenummer: 89105835.6

(22) Anmeldetag: 03.04.89

(51) Int. Cl.4: **C07B 41/04 , C07C 43/11 , C07C 41/03**

(30) Priorität: 12.04.88 DE 3812168

(43) Veröffentlichungstag der Anmeldung:
18.10.89 Patentblatt 89/42

(84) Benannte Vertragsstaaten:
**AT BE CH DE ES FR GB IT LI NL SE**

(71) Anmelder: **Henkel Kommanditgesellschaft auf Aktien**
**Postfach 1100 Henkelstrasse 67**
**D-4000 Düsseldorf 1(DE)**

(72) Erfinder: **Behler, Ansgar, Dr.**
**Siegfriedstrasse 80**
**D-4250 Bottrop(DE)**
Erfinder: **Ploog, Uwe, Dr.**
**Haydnweg 6**
**D-5657 Haan(DE)**

(54) **Verwendung von Erdalkalisalzen von Polycarbonsäuremonoestern als Katalysatoren für die Alkoxylierung.**

(57) Die Verwendung von Erdalkalisalzen von Polycarbonsäuremonoestern der allgemeinen Formel I

$$[R-(OC_mH_{2m})_n-O-CO-A(COO^-)_o]_p \ M^{2^+} \qquad (I)$$

in der

R aus der von geradkettigem oder verzweigtem $C_1$-$C_{22}$-Alkyl bzw. $C_3$-$C_{22}$-Alkenyl, Phenyl, Alkylphenyl mit 1 bis 3 $C_1$-$C_{15}$-Alkylresten, Benzyl- und Phenethyl gebildeten Gruppe,

A einen zwei- oder dreibindigen, gegebenenfalls ungesättigten acyclischen oder cyclischen Kohlenwasserstoffrest mit 2 bis 6 Kohlenstoffatomen oder einen zwei- oder dreibindigen Benzolrest,

M ein Erdalkalimetall aus der von Ca, Ba und Sr gebildeten Gruppe,

m die Zahl 2 und/oder 3,

n eine Zahl von 1 bis 20 und

o die Zahl 1 und p die Zahl 2 oder o die Zahl 2 und p die Zahl 1

bedeuten,

als Katalysatoren für die Ethoxylierung bzw. Propoxylierung von Verbindungen mit aktiven H-Atomen bietet Vorteile infolge der Löslichkeit der Katalysatoren in dem Reaktionsmedium und ergibt eine enge Homologenverteilung der Polyalkoxylierungsprodukte.

EP 0 337 239 A2

**Verwendung von Erdalkalisalzen von Polycarbonsäuremonoestern als Katalysatoren für die Alkoxylierung**

Die Erfindung betrifft die Verwendung von Erdalkalisalzen von Polycarbonsäuremonoestern der allgemeinen Formel I

$$[R\text{-}(OC_mH_{2m})_n\text{-}O\text{-}CO\text{-}A(COO^-)_o]_p \ M^{2+} \qquad (I)$$

in der

R aus der von geradkettigem oder verzweigtem $C_1$-$C_{22}$-Alkyl bzw. $C_3$-$C_{22}$-Alkenyl, Phenyl, Alkylphenyl mit 1 bis 3 $C_1$-$C_{15}$-Alkylresten, Benzyl- und Phenethyl gebildeten Gruppe,

A einen zwei- oder dreibindigen, gegebenenfalls ungesättigten acyclischen oder cyclischen Kohlenwasserstoffrest mit 2 bis 6 Kohlenstoffatomen oder einen zwei- oder dreibindigen Benzolrest,

M ein Erdalkalimetall aus der von Ca, Ba und Sr gebildeten Gruppe,

m die Zahl 2 und/oder 3 und

n eine Zahl von 1 bis 20

o die Zahl 1 und p die Zahl 2 oder o die Zahl 2 und p die Zahl 1

bedeuten,

als Katalysatoren für die Ethoxylierung bzw. Propoxylierung von Verbindungen mit aktiven H-Atomen.

Unter Verbindungen mit aktiven H-Atomen sind z.B. Fettalkohole, Fettsäuren und Amine zu verstehen, die bei der Ethoxylierung bzw. Propoxylierung nicht-ionische Detergentien bilden. Ein typisches Beispiel hierfür ist die Umsetzung von Fettalkoholen mit üblicherweise 10 bis 18 Kohlenstoffatomen mit Ethylenoxid und/oder Propylenoxid in Gegenwart von Katalysatoren, wobei die Fettalkohole mit mehreren Molekülen Ethylenoxid und/oder Propylenoxid reagieren.

Als Katalysatoren für die vorgenannte Polyalkoxylierung sind hierfür u.a. die folgenden eingesetzt worden:

Calcium- und Strontiumhydroxide, -alkoxide und phenoxide (EP-A 00 92 256),

Calciumalkoxide (EP-A 00 91 146),

Bariumhydroxid (EP-B 0 115 083),

basische Magnesiumverbindungen, z.B. Alkoxide (EP-A 00 82 569),

Magnesium- und Calciumfettsäuresalze (EP-A 0 85 167).

Die vorgenannten Katalyatoren weisen u.a. den Nachteil auf, daß sie schlecht in das Reaktionssystem einarbeitbar und/oder schwierig herstellbar sind.

Gebräuchliche Polyalkoxylierungskatalysatoren sind weiterhin Kaliumhydroxid und Natriummethylat.

Für Fettalkoholpolyalkoxylate ist eine enge Bandbreite des Polyalkoxylierungsgrades von besonderer Bedeutung, vgl. JAOCS, Vol. 63, 691 - 695 (1986), und HAPPI, 52 - 54 (1986). Die sogenannten "narrow-range"-Alkoxylate weisen demnach insbesondere die folgenden Vorteile auf:

- niedrige Fließpunkte
- höhere Rauchpunkte
- weniger Mole Alkoxid zum Erreichen der Wasserlöslichkeit
- weniger Hydrotope für das Einbringen in flüssige Universalwaschmittel
- ein geringerer, durch Anwesenheit freier (nicht umgesetzter) Fettalkohole bedingter Geruch
- Reduzierung des Plumings beim Sprühtrocknen von Waschmittelslurries, die Fettalkoholpolyalkoxylat-Tenside enthalten.

Die Bandbreite bzw. Homologenverteilung der Fettalkoholpolyalkoxylate hängt wesentlich von der Art des verwendeten Katalysators ab. Als Maß für die Homologenverteilung gilt der sogenannte Q-Wert gemäß der Beziehung

$$Q = n^* . p^2$$

in der $n^*$ die durchschnittliche Adduktzahl (mittlerer Ethoxylierungsgrad) und p den Prozentsatz des Adduktes mit bestimmten EO-Grad, das überwiegend gebildet wird, bedeuten. Ein großer Q-Wert bedeutet somit eine enge Bandbreite der Homologenverteilung.

Es wurde nun gefunden, daß man unter erfindungsgemäßer Verwendung der Erdalkalisalze von Polycarbonsäuremonoestern der obigen allgemeinen Formel I zu polyethoxylierten bzw. polypropoxylierten Fettalkoholen mit hohem Q-Wert kommt. Gegenüber den vorbekannten Katalysatoren weisen die erfindungsgemäß zu verwendenden Erdalkalisalze den Vorteil auf, daß sie kurze Reaktionszeiten ermöglichen und im Reaktionssystem, anders als z.B. Erdalkalisalze gemäß dem Stand der Technik, löslich sind.

Die den erfindungsgemäß zu verwendenden Erdalkalisalzen zugrunde liegenden Polycarbonsäuremonoester sind bekannte Verbindungen, die teilweise im Handel erhältlich sind. Sie können erhalten werden, indem man die entsprechenden Anhydride mit Alkoholen der Formel R-OH bzw. alkoxylierten Alkoholen der

2

Formel $R-(OC_mH_{2m})_n-OH$, in der R wie oben definiert ist, umsetzt und aus den so erhaltenen Polycarbonsäuremonoestern mit basischen Ca-, Ba- und Sr-Verbindungen, insbesondere den Oxiden, Hydroxiden und Carbonaten, die entsprechenden Erdalkalisalze herstellt. Die Polycarbonsäure-monoester können auch aus den freien Polycarbonsäuren durch Umsetzung mit den genannten Alkoholen bzw. alkoxylierten Alkoholen in den den Monoestern entsprechenden stöchiometrischen Mengen erhalten werden.

Gemäß einer weiteren vorteilhaften Ausführungsform der Erfindung ist das Strukturelement
$-O-CO-A(COO^-)_o$
der Polycarbonsäuremonoester der allgemeinen Formel I ein Polycarbonsäurerest aus der von Malein-, Bernstein-, Glutar, Phthal-, Dihydrophthal-, Tetrahydrophthal-, Hexahydrophthal- und Trimellithsäureresten gebildeten Gruppe. Die vorgenannten Polycarbonsäuren bilden cyclische Anhydride, die mit den Alkoholen bzw. alkoxylierten Alkoholen in molaren Verhältnissen von 1:1 in die entsprechenden Polycarbonsäuremonoester überführt werden können.

Gemäß einer weiteren vorteilhaften Ausführungsform der Erfindung ist das Erdalkalisalz der Polycarbonsäuremonoester der allgemeinen Formel I ein Erdalkalisalz eines Maleinsäurehalbesters der allgemeinen Formel II
$$[R-(OC_mH_{2m})_n-O-CO-CH=CH-COO^-]_2\ M^{2+} \qquad (II)$$
in der R, M, m und n wie oben definiert sind.

Gemäß einer vorteilhaften Ausführungsform der Erfindung werden Erdalkalisalze von Polycarbonsäuremonoestern der allgemeinen Formel I bzw. II verwendet, in denen R ein Rest aus der von geradkettigem oder verzweigtem $C_1-C_8$-Alkyl gebildeten Gruppe ist und m, n und M wie oben definiert sind, weil diese Verbindungen in Methanol löslich sind. Die höheralkylierten Derivate sind lediglich in höheren Alkoholen als Methanol löslich, können jedoch ohne weiteres auch als Alkoxylierungskatalysatoren im Sinne der Erfindung eingesetzt werden.

Bevorzugt ist weiterhin die Verwendung von Erdalkalisalzen von Polycarbonsäuremonoestern der allgemeinen Formel I bzw. II, in denen n eine Zahl von 1 bis 12 bedeutet; Verbindungen, in denen n größer als 20 wird, sind weniger interessant, weil ihre katalytische Aktivität sich vom Optimum entfernt.

Erfindungsgemäß werden die Erdalkalisalze von Polycarbonsäuremonoestern der allgemeinen Formel I bzw. II als Katalysatoren in einer Menge von 0,1 bis 2 Gew.-%, bezogen auf das Endprodukt der Ethoxylierung bzw. Propoxylierung, eingesetzt.

In den erfindungsgemäß zu verwendenden Erdalkalisalzen der Formel I bzw. II kann der Rest R z.B. eine geradkettige oder verzweigte Alkylgruppe wie Methyl, Ethyl, Propyl, n-Butyl, i-Butyl, Pentyl oder Hexyl sein, weiterhin auch eine Fettalkylgruppe wie Capryl, Caprin, Lauryl, Myristyl, Cetyl, Palmitoleyl, Oleyl, Stearoyl, Gadoleyl, Erucyl oder Behenyl. Insbesondere bei Fettalkylresten können Gemische dieser Gruppen vorliegen, wie sie z.B. bei der Umsetzung von Polycarbonsäureanhydriden mit technischen Fettalkoholen entstehen.

Die erfindungsgemäß zu verwendenden Erdalkalisalze sind flüssig bis wachsartig und in den zu alkoxylierenden Verbindungen mit aktiven H-Atomen löslich bzw. gut einarbeitbar.

Die Erfindung wird im folgenden anhand von bevorzugten Ausführungsbeispielen näher erläutert.

A. Allgemeine Herstellungsvorschrift für erfindungsgemäß einzusetzende Erdalkalisalze von Polycarbonsäuremonoestern.

Die Polycarbonsäure-monoester wurden in einem Gemisch von Wasser, Ethanol und Isopropanol (3:2:1) gelöst bzw. aufgeschlämmt und bei 90 °C mit äquimolaren Mengen Erdalkalimetallacetat umgesetzt; die Menge der zugesetzten Erdalkalimetallacetate wurde über die Säurezahl der Monoester berechnet. Die freigesetzte Essigsäure wurde mit dem Lösemittel abdestilliert.

Es wurden die in der Tabelle I zusammengefaßten Katalysatoren der allgemeinen Formel II hergestellt.

B. Ethoxylierung mit den erfindungsgemäß zu verwendenden Katalysatoren von Laurylalkohol mit 6 Mol EO.

Der Katalysator wurde in dem Laurylalkohol gelöst. Die Lösung wurde in einen für die Alkoxylierung geeigneten Autoklaven überführt. Es wurde mit Stickstoff gespült und 30 Minuten lang bei einer Temperatur von 100 °C evakuiert. Anschließend wurde die Temperatur auf 180 °C gesteigert und die gewünschte Menge Ethylenoxid bei einem Druck von 4 bis 5 bar aufgedrückt. Nach Beendigung der Reaktion ließ man 30 Minuten nachreagieren.

Die nach dieser Arbeitsvorschrift mit den erfindungsgemäß eingesetzten Katalysatoren bei der Ethoxylierung erhaltenen Ergebnisse sind in der Tabelle II zusammengefaßt. Es bedeuten

Q = der obengenannte Q-Wert.

OH-Zahl, ist/soll = OH-Zahlen der als Endprodukt erhaltenen polyethoxylierten Laurylalkohole

Kat.-% = Katalysatorkonzentration, bezogen auf das Endprodukt

t = Reaktionszeit der Polyethoxylierung.

Die mit den Katalysatoren der Formel II erhaltenen Ergebnisse sind in der Tabelle II zusammengefaßt; die Tabelle II enthält ebenfalls zu Vergleichszwecken einen Versuch mit dem als Alkoxylierungskatalysator bekannten Natriummethylat.

Der Vergleich zeigt, daß die erfindungsgemäß einzusetzenden Katalysatoren größere Q-Werte, d.h. eine bessere Homologenverteilung, als Natriummethylat ergeben.

Tabelle I

| Hergestellte Katalysatoren der allgemeinen Formel II | | | |
|---|---|---|---|
| Katalysator Nr. | R | n | M |
| 1 | $C_8$-Alkyl | 4 | Ca |
| 2 | $C_2$-Alkyl | 2 | Ca |
| 3 | $C_2$-Alkyl | 2 | Ba |
| 4 | $C_{12}$-Alkyl | 6 | Sr |
| 5 | $C_{18}/C_{18}$-Alkyl[*)] | 0 | Sr |

*) technischer Oleyl-/Stearyl-Halbester

Tabelle II

| Ethoxylierung eines handelsüblichen Laurylalkohols | | | | | |
|---|---|---|---|---|---|
| Katalysator Nr. | Kat.-% | t (h) | Q | OH-Zahl | |
| | | | | ist | soll |
| 1 | 0,5 | 7,5 | 1314 | 128 | 128,4 |
| 2 | 0,5 | 12 | 1221 | 131,9 | 122,2 |
| 3 | 0,5 | 3 | 1159 | 125,8 | 124,7 |
| 4 | 0,5 | 6,5 | 1368 | 126,3 | 126,3 |
| 5 | 0,5 | 3 | 1147 | 129,4 | 124,7 |
| NaOCH₃ | 0,5 | 4,0 | 595 | 128,8 | 126,8 |

**Ansprüche**

1. Verwendung von Erdalkalisalzen von Polycarbonsäuremonoestern der allgemeinen Formel I

$$[R-(OC_mH_{2m})_n-O-CO-A(COO^-)_o]_p \, M^{2+} \quad (I)$$

in der

R aus der von geradkettigem oder verzweigtem $C_1$-$C_{22}$-Alkyl bzw. $C_3$-$C_{22}$-Alkenyl, Phenyl, Alkylphenyl mit 1 bis 3 $C_1$-$C_{15}$-Alkylresten, Benzyl- und Phenethyl gebildeten Gruppe,

A einen zwei- oder dreibindigen, gegebenenfalls ungesättigten acyclischen oder cyclischen Kohlenwasserstoffrest mit 2 bis 6 Kohlenstoffatomen oder einen zwei-oder dreibindigen Benzolrest,

M ein Erdalkalimetall aus der von Ca, Ba und Sr gebildeten Gruppe,

m die Zahl 2 und/oder 3,

n eine Zahl von 1 bis 20 und

o die Zahl 1 und p die Zahl 2 oder o die Zahl 2 und p die Zahl 1
bedeuten,
als Katalysatoren für die Ethoxylierung bzw. Propoxylierung von Verbindungen mit aktiven H-Atomen.

2. Verwendung nach Anspruch 1, dadurch gekennzeichnet, daß das Strukturelement

$-O-CO-A(COO^-)_o$

der Polycarbonsäuremonoester der allgemeinen Formel I ein Polycarbonsäurerest aus der von Malein-, Bernstein-, Glutar-, Phthal-, Dihydrophthal, Tetrahydrophthal-, Hexahydrophthal- und Trimellithsäureresten gebildeten Gruppe ist.

3. Verwendung nach Anspruch 1 oder 2, dadurch gekennzeichnet, daß das Erdalkalisalz der Polycarbonsäuremonoester ein Erdalkalisalz eines Maleinsäurehalbesters der allgemeinen Formel II

$$[R-(OC_mH_{2m})_n-O-CO-CH=CH-COO^-]_2\ M^{2+} \qquad (II)$$

in der R, M, m und n wie oben definiert sind, ist.

4. Verwendung von Erdalkalisalzen von Polycarbonsäuremonoestern der allgemeinen Formel I bzw. II nach mindestens einen der Ansprüche 1 bis 3, in denen R ein Rest aus der von geradkettigem oder verzweigtem $C_1$- bis $C_8$-Alkyl gebildeten Gruppe ist und m, n, o, p und M wie oben definiert sind.

5. Verwendung von Erdalkalisalzen von Polycarbonsäuremonoestern der allgemeinen Formel I bzw. II nach mindestens einen der Ansprüche 1 bis 4, in denen n eine Zahl von 1 bis 12 bedeutet und R, m, o, p und M wie oben definiert sind.

6. Verwendung nach mindestens einem der Ansprüche 1 bis 5, dadurch gekennzeichnet, daß man die Katalysatoren in einer Menge von 0,1 bis 2 Gew.-%, bezogen auf das Endprodukt der Ethoxylierung bzw. Propoxylierung, einsetzt.